# EUROPÄISCHE PATENTANMELDUNG

(11) **EP 4 763 117 A1**
(43) Veröffentlichungstag der Anmeldung: **24.06.2026**
(21) Anmeldenummer: 25216581.6
(22) Anmeldetag: 18.11.2025
(51) Int. Cl.: A61B 34/00, A61B 17/29

(54) **MEDIZINISCHES INSTRUMENT**

(30) Priorität: 19.12.2024 DE 102024138988
(71) Anmelder: Karl Storz SE & Co. KG, 78532 Tuttlingen (DE)
(72) Erfinder: Mitzlaff, Lothar, 78532 Tuttlingen (DE); König, Juri, 78532 Tuttlingen (DE); Fichtner, André, 78532 Tuttlingen (DE); Silberberger, Fabian, 78532 Tuttlingen (DE); Schöntaler, Steffen, 78532 Tuttlingen (DE)

(57) **Zusammenfassung**

Die Erfindung betrifft ein medizinisches Instrument (10) umfassend:
- einen Endeffektor (12) mit einem ersten Endeffektorelement (14a) und wenigstens einem mit dem ersten Endeffektorelement (14a) zusammenwirkenden zweiten Endeffektorelement (14b);
- eine Antriebseinheit (16) mit einem ersten Antriebsrad (18a) und einem zweiten Antriebsrad (18b);
- eine Übertragungseinheit (20) mit einem drehbaren ersten Zwischenelement (22a), welches mit dem ersten Antriebsrad (18a) bewegungsübertragend gekoppelt ist, und einem drehbaren zweiten Zwischenelement (22b), welches mit dem zweiten Antriebsrad (18b) bewegungsübertragend gekoppelt ist; und
- eine Abtriebseinheit (24) mit einem drehfest mit dem ersten Endeffektorelement (14a) verbundenen ersten Verzahnungselement (26a), welches mit dem ersten Zwischenelement (22a) bewegungsübertragend gekoppelt ist, und einem drehfest mit dem zweitem Endeffektorelement (14b) verbundenen zweiten Verzahnungselement (26b), welches mit dem zweiten Zwischenelement (22b) bewegungsübertragend gekoppelt ist.

## Beschreibung

Die vorliegende Erfindung betrifft ein medizinisches Instrument.

Um die benötigte Flexibilität und/oder Gelenkigkeit medizinischer Instrumente bereitzustellen, weisen derzeit bekannte medizinische Instrumente oft einen komplexen und kostenintensiven Aufbau auf. Ein zentrales Problem aktueller Lösungen besteht darin, dass verbaute Zugseile, die für die Übertragung einer Last an Instrumentengelenken unerlässlich sind, oft über zu kleine Umlenkradien geführt werden müssen. Diese Seile sind insbesondere durch Wechselbiegung hohen mechanischen Belastungen ausgesetzt, was ihre Lebensdauer signifikant einschränkt. Die Wiederverwendbarkeit solcher Instrumente ist daher aufgrund von frühzeitiger Materialermüdung und/oder Verschleiß stark limitiert.

Um Kosten zu reduzieren, greifen viele Anwender auf Single-Use-Instrumente zurück. Diese bieten jedoch in der Regel eine eingeschränkte Anzahl an Freiheitsgraden bei der Bewegung des medizinischen Instruments, was die chirurgischen Möglichkeiten und Präzision eines behandelnden Arztes deutlich einschränkt. Ein weiteres Problem zeigt sich bei der Geometrie der Instrumente: Abwinkelungen können dazu führen, dass Hebelarme außerhalb des Querschnitts eines Rohrs oder Schafts des medizinischen Instruments positioniert sind. Dies erhöht nicht nur das Risiko von Verletzungen des Patienten, sondern kann auch das Sichtfeld eines eingesetzten Endoskops erheblich beeinträchtigen.

Der Erfindung liegt insbesondere aber nicht beschränkt darauf die Aufgabe zugrunde, ein medizinisches Instrument, insbesondere für den Einsatz in einem medizinischen Robotersystem, vorteilhaft weiterzuentwickeln, vor allem hinsichtlich einer Erhöhung einer Bewegungsfreiheit sowie einer Zuverlässigkeit. Ferner ist es unter anderem eine Aufgabe der vorliegenden Erfindung eine häufige Wiederverwendbarkeit eines solchen medizinischen Instruments zu gewährleisten und dadurch Betriebskosten zu senken.

Diese Aufgabe wird erfindungsgemäß durch die Merkmale der unabhängigen Ansprüche gelöst. Weiterbildungen der Erfindung sind den abhängigen Ansprüchen zu entnehmen.

Die Erfindung betrifft ein medizinisches Instrument umfassend:
- einen Endeffektor mit einem ersten Endeffektorelement und wenigstens einem mit dem ersten Endeffektorelement zusammenwirkenden zweiten Endeffektorelement;
- eine Antriebseinheit mit einem ersten Antriebsrad und einem zweiten Antriebsrad;
- eine Übertragungseinheit mit einem drehbaren ersten Zwischenelement, welches mit dem ersten Antriebsrad bewegungsübertragend gekoppelt ist, und einem drehbaren zweiten Zwischenelement, welches mit dem zweiten Antriebsrad bewegungsübertragend gekoppelt ist; und
- eine Abtriebseinheit mit einem drehfest mit dem ersten Endeffektorelement verbundenen ersten Verzahnungselement, welches mit dem ersten Zwischenelement bewegungsübertragend gekoppelt ist, und einem drehfest mit dem zweitem Endeffektorelement verbundenen zweiten Verzahnungselement, welches mit dem zweiten Zwischenelement bewegungsübertragend gekoppelt ist.

Durch eine derartige Ausgestaltung kann ein vorteilhaft weiterentwickeltes medizinisches Instrument bereitgestellt werden. Insbesondere kann ein solches medizinisches Instrument eine hohe Bewegungsfreiheit und Zuverlässigkeit aufweisen. Zudem kann ein Kostenaufwand für medizinische Einsätze reduziert werden, da ein derartiges medizinisches Instrument wiederverwendet werden kann.

Unter einem "medizinischen Instrument" soll ein medizinisches Werkzeug verstanden werden, das vorzugsweise dazu eingerichtet ist, ein zu bearbeitendes Objekt zu greifen, zu manipulieren, zu halten, zu schneiden und/oder anderweitig mit dem zu bearbeitenden Objekt zu interagieren. Ein zu bearbeitendes Objekt kann sich vorzugsweise auf jegliche organische und/oder anorganische Struktur beziehen. Insbesondere sind damit anatomische Strukturen eines Patienten, wie beispielsweise Organe und/oder Gewebe gemeint und/oder Verbrauchsmaterial, wie beispielsweise, Fäden, Heftklammern, Folien, Tupfer, Schläuche, Schrauben und/oder Nägel gemeint.

Das medizinische Instrument kann für den Einsatz in chirurgischen Eingriffen und/oder invasiven Operationen vorgesehen sein. Das medizinische Instrument kann Teil eines medizinischen Robotersystems und/oder zumindest mit einem solchen funktionsfähig koppelbar sein. In einigen Ausführungsformen kann das medizinische Instrument als ein handhaltbares medizinisches Instrument ausgestaltet sein. In anderen Worten kann das medizinische Instrument manuell, halbautomatisch und/oder vollautomatisch steuerbar sein.

Unter einem "Endeffektor" soll eine Komponente und/oder Vorrichtung des medizinischen Instruments verstanden werden, die vorzugsweise an einem distalen Ende des medizinischen Instruments, oder in anderen Worten, während eines Einsatzes des medizinischen Instruments, patientennah, angeordnet ist. Der Endeffektor kann dazu eingerichtet sein, physischen Kontakt mit einem und/oder mehreren zu bearbeitenden Objekten herzustellen und/oder mit diesen zu interagieren. Der Endeffektor kann je nach Anforderungsbereich und/oder Aufgabe unterschiedlich ausgebildet sein.

Das erste Endeffektorelement kann dazu eingerichtet sein, mit dem zweiten Endeffektorelement zu interagieren, zu kooperieren und/oder sich gegenseitig zu beeinflussen, um eine bestimmte Aufgabe auszuführen und/oder zu erfüllen. Das erste und das zweite Endeffektorelement können komplementär ausgebildet sein. Das erste und das zweite Endeffektorelement können einen Formschluss bilden. Die Endeffektorelemente können beispielsweise als Maulteile ausgebildet sein. Die Maulteile können dazu eingerichtet sein, die eigentliche Funktion des medizinischen Instruments auszuführen. Die Maulteile können beispielsweise zumindest als Teilkomponente einer Zange, einer Pinzette und/oder einer Schere ausgebildet sein.

Gemäß einer Weiterbildung kann das medizinische Instrument ferner einen Schaft umfassen. Bei dem Schaft kann es sich um ein längliches und/oder zylindrisches Bauteil handeln. Durch den Schaft können mechanische Komponenten und/oder Leitungen sicher und/oder geordnet geführt werden. Der Schafft kann zur Stabilität des medizinischen Instruments beitragen und/oder kinematischen Strukturen vor äußeren Einflüssen schützen.

Das erste und/oder das zweite Antriebsrad, kann/können koaxial zu einer Gelenkachse angeordnet sein, die einer Knickachse des Schafts entspricht. Durch diese bauraumsparende Ausgestaltung erhält das medizinische Instrument einen Freiheitsgrad, der es dem distalen Ende und/oder einem distalen Schaftabschnitt gestattet, sich um die Gelenkachse relativ zu einem proximalen Schaftabschnitt verschwenken zu können. Der proximaler Schaftabschnitt kann sich dabei auf einen Abschnitt des Schafts des medizinischen Instruments beziehen, der während eines Betriebs von dem Patienten abgewandt ist. Der distale Schaftabschnitt kann um zumindest 45°, bevorzugt zumindest 90° und besonders bevorzugt zumindest 160° um die Gelenkachse verschwenkbar und/oder abknickbar sein.

Das erste und/oder das zweite Antriebsrad kann/können als ein mechanisches Bauteil und/oder eine Getriebekomponente ausgebildet sein, die dazu eingerichtet ist, zumindest eine Antriebskraft und/oder zumindest ein Antriebsdrehmoment auf das erste und/oder das zweite Zwischenelement zu übertragen.

Das erste und/oder das zweite Antriebsrad kann/können als ein Zahnrad ausgebildet sein. Die Antriebsräder können vorzugsweise aus thermisch stabilen Materialien, wie Metallen und/oder Hochleistungspolymeren, wie beispielsweise Polyetherketonen, gefertigt sein. Eine derartige Ausgestaltung kann robust gegenüber Verschleiß und/oder hohen Temperaturen sein, wodurch die Lebensdauer des medizinischen Instruments, trotz Sterilisationsverfahren bei hohen Temperaturbereichen und/oder häufiger Nutzung, signifikant erhöht wird.

Das erste Endeffektorelement und/oder das zweite Endeffektorelement können unabhängig voneinander um eine Schwenkachse verschwenkbar sein, welche winklig, vorzugsweise, orthogonal zu der Gelenkachse verläuft. Das kann bedeuten, dass die beiden Endeffektorelemente separat und/oder ohne direkte Beeinflussung durch eine Bewegung des anderen Effektorelements um eine als "Schwenkachse" bezeichnete Bezugsachse verschwenkt werden können. Durch die Verschwenkbarkeit des ersten und/oder zweiten Endeffektorelements kann ein weiterer Freiheitsgrad des medizinischen Instruments geschaffen werden. Aufgrund des weiteren Freiheitsgrads kann die Bewegungsfreiheit des medizinischen Instruments und/oder die Präzision verbessert werden. Dies kann außerdem zu einer besseren Manövrierfähigkeit, einer erweiterten Reichweite und/oder einer effizienteren Durchführung von chirurgischen Eingriffen, insbesondere in schwer zugänglichen Bereichen, wie Kavitäten des Patienten, beitragen. Die Ausrichtung und/oder Steuerung des distalen Schaftabschnitts kann unabhängig von der Steuerung und/oder Ausrichtung des Endeffektors und/oder der Endeffektorelemente erfolgen.

Alternativ kann die Gelenkachse parallel zu der Schwenkachse verlaufen, um welche die Endeffektorelemente verschwenkbar sind.

Die Schwenkbewegung der Endeffektorelemente kann in vordefinierten Winkelschritten erfolgen, ist bevorzugt jedoch nahezu stufenlos und besonders bevorzugt stufenlos. Die Endeffektorelemente können jeweils um zumindest 45°, bevorzugt zumindest 90° und besonders bevorzugt zumindest 160° um die Schwenkachse verschwenkbar sein.

Gemäß einer Weiterbildung kann das erste Zwischenelement eine erste Schneckenwelle und ein erstes Zwischenzahnrad und/oder das zweite Zwischenelement eine zweite Schneckenwelle und ein zweites Zwischenzahnrad umfassen. Das erste Zwischenzahnrad kann bewegungsübertragend mit der ersten Schneckenwelle und/oder das zweite Zwischenzahnrad bewegungsübertragend mit der zweiten Schneckenwelle gekoppelt sein. Mittels einer derartigen Ausgestaltung können Bewegungen durch Abrollbewegungen zuverlässig und/oder präzise übertragen werden. Ferner kann so eine hohe Kompaktheit des medizinischen Instruments erzielt werden. Die erste und/oder die zweite Schneckenwelle können/kann eine schneckenförmige, helixförmige und/oder spiralförmige Struktur und/oder Gewinde umfassen, die/das in einen jeweiligen Wellenabschnitt oder eine jeweilige Welle eingearbeitet ist. Alternativ können/kann die erste und/oder die zweite Schneckenwelle als separate Komponente an den entsprechenden Wellenabschnitt, beispielsweise über eine Nabenverbindung, drehfest anbringbar sein.

In einigen Ausführungsformen kann das erste Zwischenzahnrad einteilig mit der ersten Schneckenwelle und/oder das zweite Zwischenzahnrad einteilig mit der zweiten Schneckenwelle ausgebildet sein. Das erste Zwischenzahnrad kann monolithisch mit der ersten Schneckenwelle und/oder das zweite Zwischenzahnrad monolithisch mit der zweiten Schneckenwelle ausgebildet sein. Eine derartige Ausgestaltung kann insbesondere eine erhöhte Stabilität und/oder Langlebigkeit gewährleisten.

Alternativ können das erste Zwischenzahnrad und die erste Schneckenwelle und/oder das zweite Zwischenzahnrad und die zweite Schneckenwelle als separate Komponenten ausgebildet sein. Diese Ausgestaltung kann Vorteile in der Herstellung, Wartung und/oder Modularität bieten, da beispielsweise beschädigte Teile leichter ersetzt und/oder die Konstruktion flexibler angepasst werden kann. Je nach Anforderungen an die Funktion, Kosten und/oder Wartungsfreundlichkeit des medizinischen Instruments, kann die Ausgestaltung der Zwischenelemente variieren.

Gemäß einer alternativen Ausführungsform kann das erste Zwischenelement statt der ersten Schneckenwelle und dem ersten Zwischenzahnrad ein erstes schrägverzahntes Zahnrad und/oder statt der zweiten Schneckenwelle und dem zweiten Zwischenzahnrad ein zweites schrägverzahntes Zahnrad umfassen. Das erste und/oder das zweite schrägverzahnte Zahnrad können vorzugsweise Zahnflanken aufweisen, die in einem Winkel, vorzugsweise einem Winkel von 45 Grad, zur Radialebene stehen. Auf diese Weise kann die Zuverlässigkeit und/oder Langlebigkeit des medizinischen Instruments erhöht werden.

Das erste Zwischenelement kann um eine erste Drehachse drehbar sein und das zweite Zwischenelement kann um eine zweite Drehachse drehbar sein. Die Zwischenelemente können derart angeordnet sein, dass die erste Drehachse und die zweite Drehachse parallel zueinander verlaufen. Die erste und/oder die zweite Drehachse kann/können winklig, vorzugsweise orthogonal, oder parallel zu der Schwenkachse angeordnet sein. Die erste und/oder die zweite Drehachse kann/können winklig, vorzugsweise orthogonal, oder parallel zu der Gelenkachse angeordnet sein.

In einigen Ausführungsformen kann die Antriebseinheit ferner eine erste Zugeinrichtung, die dazu eingerichtet ist, das erste Antriebsrad zu betätigen, und eine zweite Zugeinrichtung, die dazu eingerichtet ist, das zweite Antriebsrad zu betätigen, umfassen. Die erste und/oder die zweite Zugeinrichtung kann/können das erste und/oder zweite Antriebsrad in eine kontrollierte Rotation in beide Richtungen versetzen. Der Einsatz mehrerer Antriebseinheiten kann eine unabhängige Steuerung der einzelnen Antriebsräder gewährleisten, was zu einer erhöhten Präzision und Flexibilität in der Bewegungsausführung führt. Jedes Antriebsrad kann somit individuell geregelt werden, sodass komplexe Bewegungsabläufe, synchrone und/oder asynchrone Bewegungen präzise umgesetzt werden können.

Die erste Zugeinrichtung kann zumindest abschnittsweise, vorzugsweise größtenteils parallel zu der zweiten Zugeinrichtung verlaufen.

Die erste Zugeinrichtung und/oder die zweite Zugeinrichtung kann/können einen Seiltrieb, einen Bandtrieb, einen Riementrieb, Kettentrieb und/oder andere dem Fachmann als vorteilhaft erscheinende Getriebe, wie beispielsweise Zahnstangengetriebe, umfassen. Mittels einer derartigen Ausgestaltung können Bewegungen zuverlässig und/oder präzise über eine Distanz übertragen werden. Eine derartige Ausgestaltung kann kostengünstig in der Herstellung und/oder robust gegenüber Verschleiß sein, wodurch die Lebensdauer des medizinischen Instruments, trotz häufiger Nutzung, signifikant erhöht und die Wirtschaftlichkeit verbessert wird.

In einigen Ausführungsformen können die Bewegungen der Antriebsräder, der Zwischenelemente, und/oder der Endeffektorelemente über lineare Funktionen in Zusammenhang stehen, und diese sich bei kombinierten Bewegungen durch Addition überlagern lassen. Auf diese Weise kann eine einfache, präzise und/oder effiziente Steuerung des medizinischen Instruments realisiert werden, was zu einer insgesamt höheren Systemzuverlässigkeit führt.

Gemäß einiger Ausführungsformen kann das medizinische Instrument eine Schnelllösevorrichtung umfassen, die in einem arretierten Zustand dazu eingerichtet ist, die Endeffektorelemente gegen eine axiale und/oder radiale Verschiebung relativ zu der Schwenkachse zu sichern und die in einem gelösten Zustand dazu eingerichtet ist, eine axiale und/oder radiale Verschiebung der Endeffektorelemente relativ zu der Schwenkachse zu erlauben. Die Schnelllösevorrichtung kann insbesondere bei medizinischen Instrumenten von Vorteil sein, deren Endeffektorelemente sich sehr schnell abnutzen, wie beispielsweise Schneidklingen bei Schnittinstrumenten. Eine solche Schnelllösevorrichtung kann den Wechsel der Endeffektorelemente erleichtern, da die Schnelllösevorrichtung durch einen einfachen Mechanismus, wie zum Beispiel einen Hebel, Knopf und/oder Drehverschluss, schnell und vorzugsweise werkzeuglos geöffnet werden kann. Dies kann die Benutzerfreundlichkeit und Sicherheit erhöhen, da der Wechsel ohne direktes Berühren der Endeffektorelemente erfolgen kann.

Die erfindungsgemäßen Vorrichtungen sollen hierbei nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Im Folgenden wird die vorliegende Erfindung anhand der beigefügten Figuren beispielhaft beschrieben. Die Zeichnungen, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine perspektivische Ansicht eines medizinischen Instruments mit einer kinematischen Struktur;
- Fig. 2: eine Detailansicht der kinematischen Struktur des medizinischen Instruments in einer ersten Position;
- Fig. 3: eine weitere Detailansicht der kinematischen Struktur des medizinischen Instruments in der ersten Position;
- Fig. 4: eine Detailansicht der kinematischen Struktur des medizinischen Instruments in einer zweiten Position;
- Fig. 5: eine Detailansicht einer Hälfte der kinematischen Struktur des medizinischen Instruments;
- Fig. 6: eine perspektivische Ansicht eines Zwischenelements des medizinischen Instruments; und
- Fig. 7: eine Detailansicht einer kinematischen Struktur eines medizinischen Instruments gemäß einer weiteren Ausführungsform.

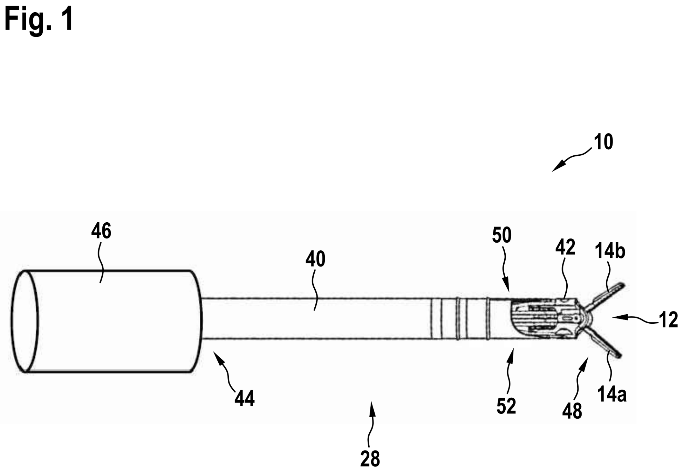

In Fig. 1 ist ein medizinisches Instrument 10 mit einem Schaft 28 gezeigt. Der Schaft 28 weist einen proximalen Schaftabschnitt 40 und einen distalen Schaftabschnitt 42 auf. An einem proximalem Ende 44 des proximalen Schaftabschnitts 40 ist eine Schnittstelle 46 des medizinischen Instruments 10 angeordnet. Die Schnittstelle 46 kann dazu eingerichtet sein, mit einer hierin nicht gezeigten Robotervorrichtung und/oder einer Handhabe funktionsfähig und/oder steuerbar gekoppelt zu werden. Der Schaft kann um eine Rotationsachse R rotierbar sein. An einem distalen Ende 50 des proximalen Schaftabschnitts 40 ist der distale Schaftabschnitt 42 des medizinischen Instruments 10 mit einem Endeffektor 12 angeordnet. Der Endeffektor 12 umfasst eine Greifanordnung 48 mit zwei Endeffektorelementen 14a, 14b. Die Greifanordnung 48 ist in einer offenen Greifposition gezeigt.

An dieser Stelle sei darauf hingewiesen, dass die Greifanordnung 48 lediglich beispielhaft zu verstehen ist und je nach Anwendungsbereich auch andere dem Fachmann vorteilhaft erscheinende Endeffektoren 12 und/oder Anordnungen vorgesehen seien können. Eine Länge des proximalen Schaftabschnitts 40 kann insbesondere zumindest dem fünffachen, vorzugsweise zumindest dem zehnfachen und besonders bevorzugt zumindest dem fünfzehnfachen der Länge des distalen Schaftabschnitts 42 entsprechen.

Der Endeffektor 12, die Endeffektorelemente 14a, 14b und/oder der distale Schaftabschnitt 42 lassen sich mittels einer kinematischen Struktur 52 des medizinischen Instruments 10 steuern und/oder bewegen.

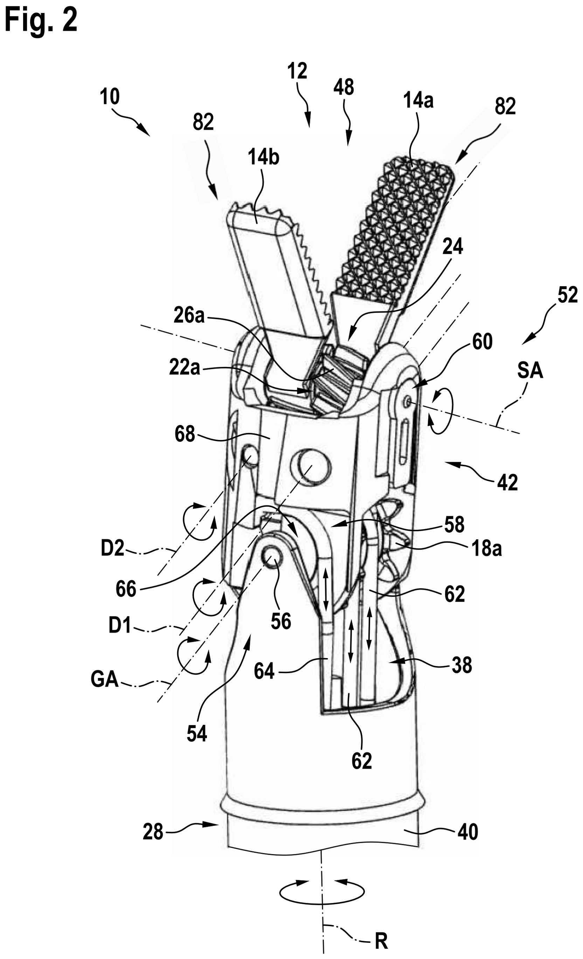

In Fig. 2 ist eine Detailansicht der kinematischen Struktur 52 des medizinischen Instruments 10 in einer ersten Position gezeigt. Zu erkennen ist das distale Schaftende 54 des proximalen Schaftabschnitts 40, welches mittels eines ersten Lagerbolzens 56 verliersicher und beweglich mit einem proximalen Schaftende 58 des distalen Schaftabschnitts 42 gekoppelt ist. Der distale Schaftabschnitt 42 ist derart ausgebildet, dass die längste Erstreckung dessen Querschnitts nicht über den Querschnitt des proximalen Schaftabschnitts 40 ragt. Dadurch können ungewollte Bewegungen und/oder das Verletzungsrisiko eines Patienten während einer Behandlung durch einen Arzt reduziert werden. Ferner kann das Sichtfeld des behandelnden Arztes durch eine derartige Ausgestaltung des medizinischen Instruments 10 weniger eingeschränkt.

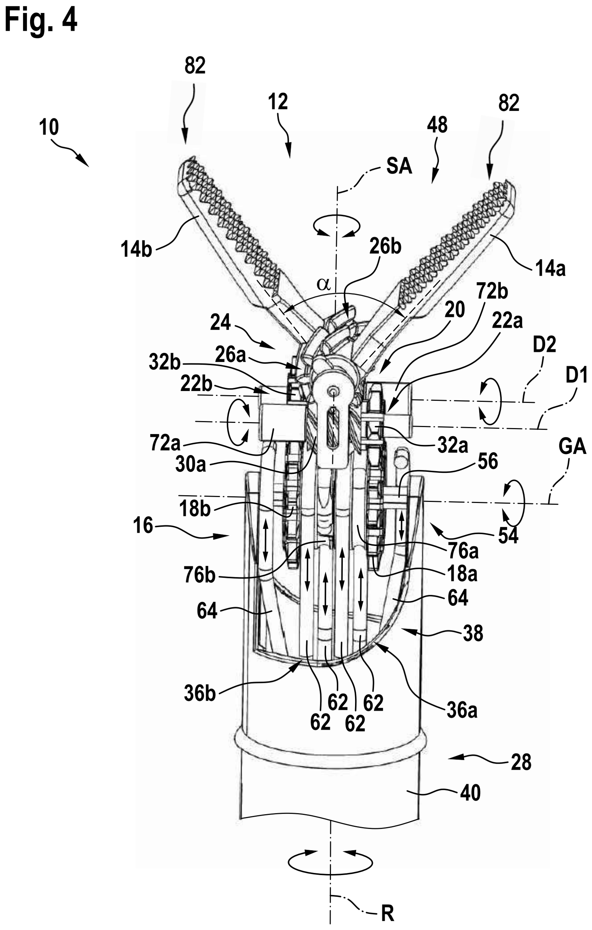

Der Lagerbolzen 56 ist gemäß Fig. 1 in einer Wandung 68 des distalen Schaftabschnitts 42 schwenkbar und/oder feststehend gelagert. Der distale Schaftabschnitt 42 weist zwei Kopplungsabschnitte 66 auf, an denen er über den Lagerbolzen 56 mit dem proximalen Schaftabschnitt 40 gelenkig gekoppelt ist. In der vorliegenden Fig. 1 ist lediglich ein Kopplungsabschnitt 66 gezeigt. Der andere Kopplungsabschnitt 66 befindet sich auf der gegenüberliegenden Seite des distalen Schaftabschnitts 42, also auf der Seite, die auf der Rückseite der Zeichenebene der Fig.1 liegt. Die Kopplungsabschnitte 66 sind dazu eingerichtet jeweils mit einem Schwenkseil 64 eines Seiltriebs 38 bewegungs- und/oder kraftübertragend gekoppelt zu werden. Für eine bessere Ansicht des Seiltriebs kann auf die Fig. 4 verwiesen werden. Je nachdem welches der Schwenkseile 64 betätigt und/oder mit Zug belastet wird, schwenkt der distale Schaftabschnitt 42 in die eine oder in die andere Richtung um eine Gelenkachse GA, die vorzugsweise koaxial zu einer Längsachse des ersten Lagerbolzens 56 verläuft. Wird eines der beiden Schwenkseile 64 auf Zug beansprucht so wird das beanspruchte Schwenkseil 64 vom Kopplungsabschnitt 66 aufgerollt und das andere Schwenkseil 64 auf dem anderen Kopplungsabschnitt 66 aufgerollt. Die Kopplungsabschnitte 66 und/oder die Schwenkseile 64 können interdependent gekoppelt sein. Das bedeutet, dass die Kopplungsabschnitte und/oder die Schwenkseile 64 sich insbesondere hinsichtlich ihres Wickelwinkels gegenseitig beeinflussen. Dabei kann der Wickelwinkel und/oder das Verhalten eines jeden Schwenkseils 64 von dem Wickelwinkel und/oder dem Verhalten eines jeden Schwenkseils 64 abhängig sein.

Auf dem Lagerbolzen 56 ist eine Antriebseinheit 16 mit zwei Antriebsrädern 18a, 18b angeordnet. Die Antriebsräder 18a, 18b sind über eine jeweilige Seilscheibe 76a, 76b bewegungsübertragend mit Antriebsseilen 62 gekoppelt. Sowohl die Antriebsräder 18a, 18b als auch die Seilscheiben sind koaxial auf dem ersten Lagerbolzen 56 angeordnet. Die Seilscheiben 76a, 76b sind jeweils mit zwei Antriebsseilen 62 gekoppelt. Je nachdem welche Seilscheibe 62 betätigt wird, wird das erste Antriebsrad 18a und/oder das zweite Antriebsrad 18b in eine Drehbewegung um den ersten Lagerbolzen 56 und/oder die Gelenkachse GA versetzt. Je nachdem welches der beiden mit einer der Seilscheiben gekoppelten Antriebsseile 62 betätigt wird, wird das entsprechende Antriebsrad 18a, 18b in die eine oder in die andere Richtung um den ersten Lagerbolzen 56 und/oder die Gelenkachse GA gedreht. Die Seilscheiben 62 und/oder die Antriebsräder 18a, 18b sind zumindest abschnittsweise innerhalb des distalen Schaftabschnitts 42 angeordnet. Das erste Antriebsrad 18a ist unabhängig von dem zweiten Antriebsrad 18b bei Betätigung eines entsprechenden Antriebsseils 62 um die Gelenkachse GA und/oder den Lagerbolzen 56 drehbar.

Die Antriebsräder 18a und 18b sind über eine Übertragungseinheit 20 bewegungsübertragend mit dem Endeffektor 12 gekoppelt. Der Endeffektor 12 ist an einem distalen Bereich des distalen Schaftabschnitts 42 angeordnet und weist die beiden Endeffektorelemente 14a, 14b auf. Die beiden Endeffektorelemente 14a, 14b sind in der hierin gezeigten Ausführungsform jeweils als ein Maulteil 82 konfiguriert. Die beiden Endeffektorelemente 14a, 14b sind komplementär ausgebildet, sodass sie mit dem jeweils anderen Endeffektorelement 14a, 14b zusammenwirken können. Die beiden Endeffektorelemente 14a, 14b sind unabhängig voneinander um eine Schwenkachse SA verschwenkbar. Die beiden Endeffektorelemente 14a, 14b sind in der in den Figuren 1 bis 5 gezeigten Position in einem Winkel α relativ zueinander um die Schwenkachse SA verschwenkt.

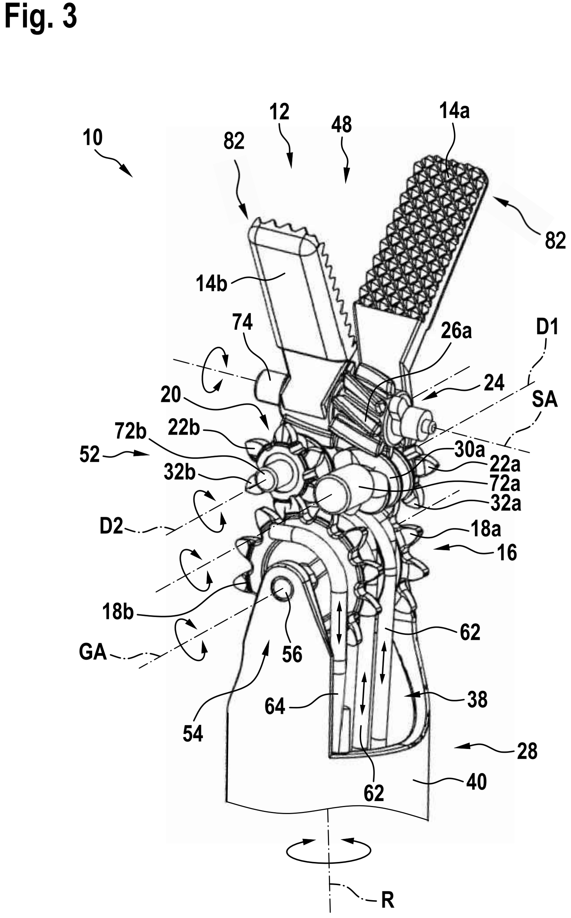

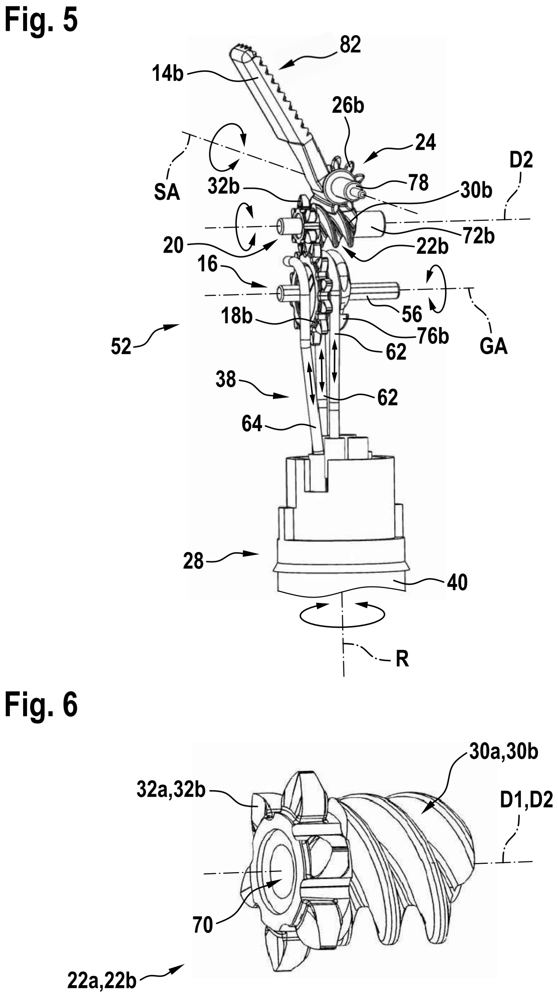

Für weitere Details der kinematischen Struktur 52 kann auf die Figuren 3 bis 6 verwiesen werden. In diesen Figuren ist die Wandung 68 ausgeblendet. Zu erkennen ist die Übertragungseinheit 20, die dazu eingerichtet ist, Bewegungen und/oder Kräfte von den Antriebsrädern 18a, 18b auf die Abtriebseinheit 24 und/oder die Endeffektorelemente 14a, 14b zu übertragen. Die Übertragungseinheit 20 umfasst zwei Zwischenelemente 22a, 22b, die in der hierin gezeigten Ausführungsform eine erste und eine zweite Schneckenwelle 30a, 30b umfassen. Das erste Zwischenelement 22a ist um eine Drehachse D1 und das zweite Zwischenelement um eine Drehachse D2 drehbar. Die Schneckenwellen 30a, 30b sind vorliegend als Hohlwellen ausgebildet. Jede der beiden Schneckenwellen 30a, 30b weist an einer Stirnseite ein Zwischenzahnrad 32a, 32b auf. Ein derartiges Zwischenelement 22a, 22b ist isoliert in Fig. 6 gezeigt. Das Zwischenzahnrad 32a, 32b ist einteilig und/oder monolithisch mit der Schneckenwelle 30a, 30b ausgebildet. Ferner weist jedes der Zwischenelemente 22a, 22b eine Öffnung 70 auf. Über die Öffnung 70 können die Zwischenelemente 22a, 22b jeweils auf einen Anschlagbolzen 72a, 72b der Übertragungseinheit 20 geführt werden. Jeder der Anschlagbolzen 72a, 72b ist wie auch der erste Lagerbolzen 78 in der Wandung 68 gelagert. Die Anschlagbolzen 72a, 72b weisen jeweils einen Anschlag auf, der eine Verschiebung der Zwischenelemente 22a, 22b in axialer Richtung und/oder entlang der Anschlagbolzen 72a, 72b verhindert.

Die Anschlagbolzen 72a, 72b und/oder die Zwischenelemente 22a, 22b sind vorzugsweise parallel zu der Gelenkachse GA und/oder dem ersten Lagerbolzen 56 angeordnet. Die Anschlagbolzen 72a, 72b und die Zwischenelemente 22a, 22b sind über dem Lagerbolzen 56 angeordnet. Ferner sind das erste Zwischenelement 22a und das zweite Zwischenelement 22b spiegelverkehrt und hintereinander angeordnet. Eine derartige Bauweise ermöglicht es, den Bauraum besonders effizient zu nutzen und das medizinische Instrument 10 kompakt zu gestalten.

Das erste Zwischenelement 22a, ist über das erste Zwischenzahnrad 32a bewegungsübertragend mit dem ersten Antriebsrad 18a gekoppelt. Das zweite Zwischenelement 22b, ist über das zweite Zwischenzahnrad 32b bewegungsübertragend mit dem zweiten Antriebrad 18b gekoppelt. Zur bewegungsübertragenden Kopplung greift eine Verzahnung des ersten Zwischenzahnrads 32a in eine Verzahnung des ersten Antriebsrads 18a und Verzahnung des zweiten Zwischenzahnrads 32b greift in eine Verzahnung des zweiten Antriebsrads 18b. Die Verzahnungen der Zwischenzahnräder 32a, 32b und die der Antriebsräder 18b sind komplementär ausgebildet.

Zugleich ist die Übertragungseinheit 20 auch mit der Abtriebseinheit 24 gekoppelt. Dabei greift ein erstes Verzahnungselement 26a und/oder zumindest ein Teil dessen Verzahnung zumindest abschnittsweise in ein Schneckengewinde der ersten Schneckenwelle 30a. Analog greift ein zweites Verzahnungselement 26b und/oder zumindest ein Teil dessen Verzahnung zumindest abschnittsweise in ein Schneckengewinde der zweiten Schneckenwelle 30b. Die Verzahnungselemente 26a, 26b sind in der hierin gezeigten Ausführungsform einteilig mit dem jeweiligen Endeffektorelement 14a, 14b ausgebildet. Die Endeffektorelemente sind mit dessen jeweiligem Endabschnitt auf einem Anschlagstift 74 oder einem zweiten Lagerbolzen 78 angeordnet. Der zweiter Lagerbolzen 78 und/oder der Anschlagstift 74 sind orthogonal zu der Gelenkachse GA und den Drehachsen D1 und D2 angeordnet.

Die Gelenkachse GA, die Schwenkachse SA, die Rotationsachse R sowie die Drehachsen D1 und D2 sind in den Figuren durch gestrichelte Linien angedeutet. Durch die voranstehenden Achsen erlangten Freiheitsgrade der Antriebsräder, 18a, 18b, der Zwischenelemente 22a, 22b, der Endeffektorelemente 14a, 14b und/oder des Schafts 28, sind als Pfeile angedeutet. Jedes der gezeigten medizinischen Instrumente kann sechs Freiheitsgrade aufweisen.

Um einen flexiblen Einsatz sowie eine hohe Wiederverwendbarkeit des medizinischen Instruments 10 zu gewährleisten, weist das medizinische Instrument eine Schnelllösevorrichtung 20 auf. Diese Schnelllösevorrichtung kann beispielsweise einen mechanischen Spann- und/oder Klemmmechanismus umfassen. Dieser Mechanismus kann beispielsweise durch Betätigen eines Hebels, Drückens eines Knopfes und/oder Drehen eines Riegels geöffnet werden, wodurch die Endeffektorelemente 14a, 14b freigegeben werden. Zum Fixieren werden die neuen Endeffektorelemente 14a, 14b auf den vorgesehenen zweiten Lagerbolzen 78 oder Anschlagbolzen 72a, 72b geschoben, und die Schnelllösevorrichtung 60 kann die Endeffektorelemente 14a, 14b beispielsweise durch Reibung, Druck und/oder einen Formschluss sicher an Ort und Stelle arretieren.

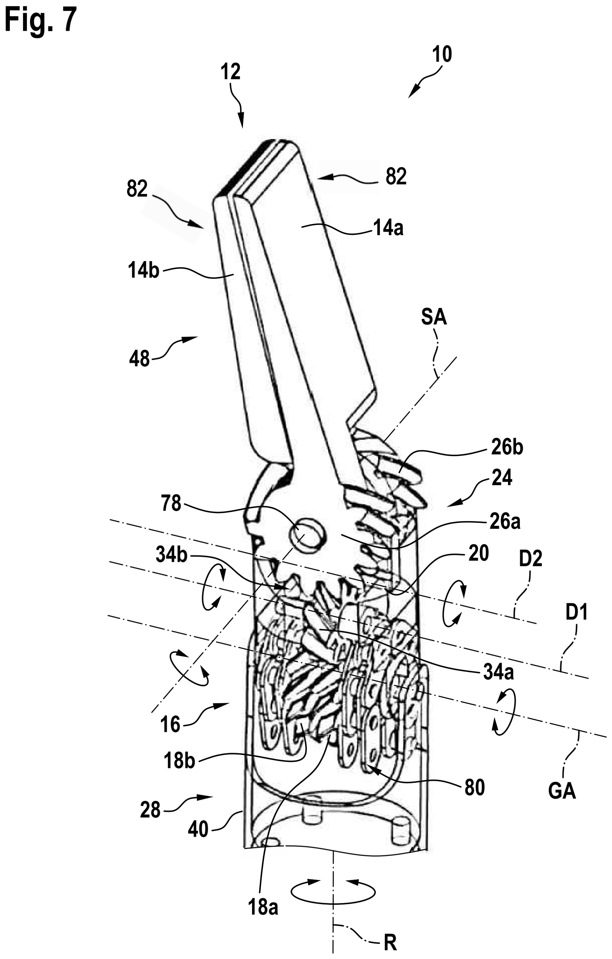

In Figur 7 ist eine weitere Ausführungsform eines medizinischen Instruments gezeigt. Die in Fig. 7 gezeigte Ausführungsform unterscheidet sich von der in den Figuren 1 bis 5 dargestellten insofern, dass in Fig. 7 die Seiltriebe 38 durch Kettentriebe 80 ersetzt sind. Ferner sind die Zwischenelemente 22a, 22b nicht als Schneckenwellen 30a, 30b und Zwischenzahnräder 32a, 32b sondern als schrägverzahnte Zahnräder 34a, 34b ausgebildet. Die Greifanordnung 48 ist außerdem im Gegensatz zu den Figuren 1 bis 5 in einer geschlossenen Greifposition gezeigt.

### Bezugszeichenliste

- 10: Medizinisches Instrument
- 12: Endeffektor
- 14a,b: Endeffektorelement
- 16: Antriebseinheit
- 18a,b: Antriebsrad
- 20: Übertragungseinheit
- 22a,b: Zwischenelement
- 24: Abtriebseinheit
- 26a,b: Verzahnungselement
- 28: Schaft
- 30a,b: Schneckenwelle
- 32a,b: Zwischenzahnrad
- 34a,b: schrägverzahntes Zahnrad
- 36a,b: Zugeinrichtung
- 38: Seiltrieb
- 40: proximaler Schaftabschnitt
- 42: distaler Schaftabschnitt
- 44: proximales Ende
- 46: Schnittstelle
- 48: Greifanordnung
- 50: distales Ende
- 52: kinematische Struktur
- 54: distales Schaftende
- 56: erster Lagerbolzen
- 58: proximales Schaftende
- 60: Schnelllösevorrichtung
- 62: Antriebsseil
- 64: Schwenkseil
- 66: Kopplungsabschnitt
- 68: Wandung
- 70: Öffnung
- 72a,b: Anschlagbolzen
- 74: Anschlagstift
- 76a,b: Seilscheibe
- 78: zweiter Lagerbolzen
- 80: Kettentrieb
- 82: Maulteil
- α: Winkel
- GA: Gelenkachse
- R: Rotationsachse
- SA: Schwenkachse
- D1: erste Drehachse
- D2: zweite Drehachse

## Patentansprüche

1. Medizinisches Instrument (10) umfassend:
- einen Endeffektor (12) mit einem ersten Endeffektorelement (14a) und wenigstens einem mit dem ersten Endeffektorelement (14a) zusammenwirkenden zweiten Endeffektorelement (14b);
- eine Antriebseinheit (16) mit einem ersten Antriebsrad (18a) und einem zweiten Antriebsrad (18b);
- eine Übertragungseinheit (20) mit einem drehbaren ersten Zwischenelement (22a), welches mit dem ersten Antriebsrad (18a) bewegungsübertragend gekoppelt ist, und einem drehbaren zweiten Zwischenelement (22b), welches mit dem zweiten Antriebsrad (18b) bewegungsübertragend gekoppelt ist; und
- eine Abtriebseinheit (24) mit einem drehfest mit dem ersten Endeffektorelement (14a) verbundenen ersten Verzahnungselement (26a), welches mit dem ersten Zwischenelement (22a) bewegungsübertragend gekoppelt ist, und einem drehfest mit dem zweitem Endeffektorelement (14b) verbundenen zweiten Verzahnungselement (26b), welches mit dem zweiten Zwischenelement (22b) bewegungsübertragend gekoppelt ist.

2. Medizinisches Instrument (10) nach Anspruch 1, ferner umfassend:
- einen Schaft (28), wobei das erste und/oder das zweite Antriebsrad (18a, 18b), koaxial zu einer Gelenkachse (GA) angeordnet sind/ist, die einer Knickachse des Schafts (28) entspricht.

3. Medizinisches Instrument (10) nach Anspruch 2, wobei das erste Endeffektorelement (14a) und/oder das zweite Endeffektorelement (14b) unabhängig voneinander um eine Schwenkachse (SA) verschwenkbar sind, welche orthogonal zu der Gelenkachse (GA) verläuft.

4. Medizinisches Instrument (10) nach einem der vorherigen Ansprüche, wobei das erste Zwischenelement (22a) eine erste Schneckenwelle (30a) und ein erstes Zwischenzahnrad (32a) und/oder das zweite Zwischenelement (22b) eine zweite Schneckenwelle (30b) und ein zweites Zwischenzahnrad (32b) umfasst, wobei das erste Zwischenzahnrad (32a) bewegungsübertragend mit der ersten Schneckenwelle (30a) und/oder das zweite Zwischenzahnrad (32b) bewegungsübertragend mit der zweiten Schneckenwelle (30b) gekoppelt sind/ist.

5. Medizinisches Instrument (10) nach Anspruch 4, wobei das erste Zwischenzahnrad (32a) einteilig mit der ersten Schneckenwelle (30a) und/oder das zweite Zwischenzahnrad (32b) einteilig mit der zweiten Schneckenwelle (30b) ausgebildet ist.

6. Medizinisches Instrument (10) nach einem der Ansprüche 1 bis 3, wobei das erste Zwischenelement (22a) ein erstes schrägverzahntes Zahnrad (34a) und/oder das zweite Zwischenelement (22b) ein zweites schrägverzahntes Zahnrad (34b) umfasst.

7. Medizinisches Instrument (10) nach einem der vorherigen Ansprüche, wobei die Antriebseinheit (16) ferner eine erste Zugeinrichtung (36a), die dazu eingerichtet ist, das erste Antriebsrad (18a) zu betätigen, und eine zweite Zugeinrichtung (36b), die dazu eingerichtet ist, das zweite Antriebsrad (18b) zu betätigen, umfasst.

8. Medizinisches Instrument (10) nach Anspruch 7, wobei die erste Zugeinrichtung (36a) und/oder die zweite Zugeinrichtung (36b) einen Seiltrieb (38), einen Bandtrieb, einen Riementrieb und/oder einen Kettentrieb umfasst.

9. Medizinisches Instrument (10) nach einem der vorherigen Ansprüche, wobei Bewegungen der Antriebsräder (18a, 18b), der Zwischenelemente (22a, 22b), und/oder der Endeffektorelemente (14a, 14b) über lineare Funktionen in Zusammenhang stehen, und diese sich bei kombinierten Bewegungen durch Addition überlagern lassen.

10. Medizinisches Instrument (10) nach einem der Ansprüche 3 bis 9, ferner umfassend:
- eine Schnelllösevorrichtung (60), die in einem arretierten Zustand dazu eingerichtet ist, die Endeffektorelemente (14a, 14b) gegen eine axiale und/oder radiale Verschiebung relativ zu der Schwenkachse (SA) zu sichern und die in einem gelösten Zustand dazu eingerichtet ist, eine axiale und/oder radiale Verschiebung der Endeffektorelemente (14a, 14b) relativ zu der Schwenkachse (SA) zu erlauben.
